(19) Europäisches Patentamt
European Patent Office
Office européen des brevets

(11) Veröffentlichungsnummer: **0 002 480**
**B1**

# (12) EUROPÄISCHE PATENTSCHRIFT

(45) Veröffentlichungstag der Patentschrift: **09.06.82**

(21) Anmeldenummer: **78101540.9**

(22) Anmeldetag: **04.12.78**

(51) Int. Cl.³: **C 07 D 261/08,**
C 07 C 135/00

## (54) Verfahren zur Herstellung von 1,2-Oxazolderivaten.

(30) Priorität: **09.12.77 DE 2754832**

(43) Veröffentlichungstag der Anmeldung:
**27.06.79 Patentblatt 79/13**

(45) Bekanntmachung des Hinweises auf die Patenterteilung:
**09.06.82 Patentblatt 82/23**

(84) Benannte Vertragsstaaten:
**BE CH DE FR GB IT**

(56) Entgegenhaltungen:
**FR - A - 1 548 997**
**GB - A - 949 372**
**US - A - 3 213 068**

(73) Patentinhaber: **BASF Aktiengesellschaft**
**Carl-Bosch-Strasse 38**
**D-6700 Ludwigshafen (DE)**

(72) Erfinder: **Theobald, Hans, Dr.**
**Parkstrasse 2**
**D-6703 Limburgerhof (DE)**

**EP 0 002 480 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von 1,2-Oxazolderivaten

Die Erfindung betrifft ein Verfahren zur Herstellung von 1,2-Oxazolderivaten durch Umsetzung von Propargylhalogenid oder -alkohol mit in situ hergestellten aliphatischen Nitriloxiden.

Es ist bekannt, daß 1,2-Oxazolderivate, die in 3-Stellung einen aliphatischen Substituenten tragen, durch Umsetzung von Nitriloxiden mit Propargylderivaten erhalten werden können. Die als Ausgangsstoffe verwendeten aliphatischen Nitriloxide werden dabei durch Dehydrohalogenierung von Hydroxamsäurehalogeniden oder durch Wasserabspaltung aus aliphatischen Nitroverbindungen erhalten (DE—A—25 49 962).

Weiterhin ist bekannt, daß aromatische und heterocyclische Nitriloxide durch Dehydrierung des Alkalisalzes des entsprechenden Aldoxims mit Hypohalogenit in wäßriger Phase hergestellt werden können (Fortsch. Chem. Forsch. 7, 68 (1966)). Dabei ergibt die Umsetzung mit Natriumhypobromit Nitriloxide in guten Ausbeuten, während die Umsetzung mit Natriumhypochlorit im wesentlichen unter Dimerisierung von dehydrierten Aldoximmolekeln zu Aldazin-bis-oxiden führt (J. Org. Chem. *30*, 2809 ff (1965); Chem. Ber. *61*, 290 ff. (1961)).

Die Umsetzung mit Hypochlorit führt nur dann in guten Ausbeuten zum aromatischen Nitriloxid, wenn am Aromaten substituierte Aldoxime verwendet werden (GB—A—949 372).

Als mögliches Herstellungsverfahren für aliphatische Nitriloxide wird in der FR—A—1 548 937, die die Herstellung von Nitrilsulfitaddukten betrifft, die Umsetzung der entsprechenden Oxime mit Hypohalogenit erwähnt, beschrieben wird die Durchführung des Verfahrens jedoch nur für aromatische Nitriloxide.

Es wurde ein Verfahren zur Herstellung von 1,2-Oxazolderivaten durch Umsetzung von Propargylhalogenid oder -alkohol mit in situ hergestellten aliphatischen Nitriloxiden der Formel I

$$R—C{\equiv}N{\rightarrow}O \qquad (I),$$

in der R für einen unverzweigten oder verzweigten, gesättigten aliphatischen Rest steht, der einen oder mehrere Substituenten tragen kann, die nicht mit Hypochlorit reagieren, gefunden, das dadurch gekennzeichnet ist, daß das verwendete Nitriloxid durch Umsetzung eines Aldoxims der Formel II

$$R—CH{=}NOH \qquad (II),$$

in der R die oben genannte Bedeutung hat, mit Hypochlorit in Gegenwart eines Lösungsmittels bei Temperaturen im Bereich zwischen −80°C und dem Siedepunkt des Lösungsmittels hergestellt wird und das in situ gebildete Nitriloxid der Formel I zu einem 1,2-Oxazolderivat umgesetzt wird.

Durch Hypochlorit werden aliphatische Aldoxime mit sehr hohen Ausbeuten zu Nitriloxiden dehydriert, während Hypobromit unter gleichen Reaktionsbedingungen nur zu geringen Mengen an nachweisbarem Nitriloxid führt. Dieses unterschiedliche Verhalten von Hypobromit und Hypochlorit gegenüber aliphatischen Aldoximen war keineswegs zu erwarten, da in Fortsch. Chem. Forsch. 7, 68 (1966) darauf hingewiesen wird, daß die Umsetzung mit Natriumhypobromit auch auf aliphatische Aldoxime übertragbar ist.

Bei dem erfindungsgemäßen Verfahren können in situ hergestellte Nitriloxide der Formel I eingesetzt werden, bei denen R für einen unverzweigten oder verzweigten, gesättigten aliphatischen Rest steht, der einen oder mehrere Substituenten tragen kann, die nicht mit Hypochlorit reagieren. In Betracht kommen unverzweigte oder verzweigte Alkylreste mit 1 bis 15 Kohlenstoffatomen, vorzugsweise mit 1 bis 10, insbesondere mit 1 bis 4 Kohlenstoffatomen, Alkoxyalkylreste mit 2 bis 10 Kohlenstoffatomen, vorzugsweise 2 bis 6 Kohlenstoffatomen, insbesondere 2 bis 4 Kohlenstoffatomen, Halogenalkylreste mit einem oder mehreren Halogensubstituenten und 1 bis 10 Kohlenstoffatomen, vorzugsweise 1 bis 6 Kohlenstoffatomen, insbesondere 1 bis 4 Kohlenstoffatomen, wobei Halogen Fluor, Chlor, Brom oder Jod sein kann, oder Phenylalkylreste, wobei der Phenylrest gegebenenfalls durch Alkyl mit 1 bis 4 Kohlenstoffatomen, Alkoxy mit 1 bis 4 Kohlenstoffatomen, Halogen, Nitro oder Cyano substituiert sein kann.

Beispiele für die für R stehenden Reste sind Alkylreste, wie Methyl, Äthyl, Propyl, i-Propyl, n-Butyl, i-Butyl, sec-Butyl, tert.-Butyl, n-Pentyl, iso-Pentyl, die Hexyl-, Heptyl-, Octyl-, Nonyl- und Decyl-Reste, Methoxyalkylreste, wie Methoxymethyl, 1-Methoxyäthyl, 2-Methoxyäthyl, 3-Methoxy-n-propyl, 2-Methoxy-n-propyl, 4-Methoxy-n-Butyl, Äthoxyalkylreste, wie Äthoxymethyl, 1-Äthoxyäthyl, 2-Äthoxyäthyl, 2-Äthoxy-n-propyl, 4-Äthoxy-n-butyl, Isopropyloxyalkylreste, wie Isopropyloxymethyl, 2-Isopropyloxyäthyl, Fluoralkylreste, wie Fluormethyl, Trifluormethyl, Difluormethyl, Chloralkylreste, wie Chlormethyl, 1-Chloräthyl, 2-Chloräthyl, Bromalkylreste, wie Brommethyl, Jodalkylreste, wie Jodmethyl, oder durch Phenyl substituierte Alkylreste, wie Benzyl, 1-Phenyläthyl, 2-Phenyläthyl.

Die unter Verwendung aliphatischer Nitriloxide der Formel I hergestellten 1,2-Oxazolderivate sind wertvolle Zwischenprodukte für die Herstellung biologisch aktiver Verbindungen, wie Insektizide (DE—A—25 49 961) und Herbizide (DE—A—26 33 790).

Bei der Herstellung der aliphatischen Nitriloxide kann Hypochlorit in Form von Alkalimetallhypochloriten, Erdalkalimetall-hypochloriten, tert.-Butylhypochlorit oder hypochloriger Säure in wäßriger Lösung eingesetzt werden. Als Alkalimetall- und Erdalkalimetallhypochlorite kommen Natriumhypochlorit, Kaliumhypochlorit, Calciumhypochlorit, Magnesiumhypochlorit, Bariumhypochlorit oder Strontiumhypochlorit in Betracht. Die Hypochlorite können ebenfalls in situ im Reaktionsgefäß durch Einwirken von Chlor auf Alkalihydroxide, -hydrogencarbonate und -carbonate erzeugt werden. Bevorzugt verwendet man die handelsüblichen technischen wäßrigen Lösungen der Hypochlorite.

Geeignete Lösungsmittel, in denen die Umsetzung von Aldoxim mit Hypochlorit und von so hergestelltem Nitriloxid mit Propargylhalogenid oder -alkohol durchgeführt werden kann, sind beispielsweise Wasser; Alkohole, wie Methanol, Äthanol, Propanol, Isopropanol; Ketone, wie Aceton, Methyläthylketon; Äther, wie Dimethyläther, Diäthyläther, Tetrahydrofuran; chlorierte aliphatische Kohlenwasserstoffe, wie Methylenchlorid, Chloroform, Tetrachlorkohlenstoff, Dichloräthan; aromatische Verbindungen, wie Benzol, Toluol, Xylole, Chlorbenzole; Ester wie Essigester; Dimethylformamid; Dimethylsulfoxid. Ebenso kann die zum Abfangen des Nitriloxids vorhandene Verbindung als Lösungsmittel dienen. Bevorzugtes Lösungsmittel ist Wasser.

Die Umsetzung kann sowohl in einem Einphasen- als auch in einem Mehrphasensystem durchgeführt werden. Bei Zwei- oder Mehrphasensystemen können Phasentransferkatalysatoren benutzt werden. Solche Phasentransferkatalysatoren sind beispielsweise substituierte Ammoniumsalze, wie Triäthylbenzylammoniumchlorid, Trimethylbenzylammoniumbromid, Triphenylbenzylammoniumchlorid, Methyltributylammoniumjodid oder Tetrabutylammoniumhydrogensulfat, oder substituierte Phosphoniumsalze, wie Benzyltributylphosphoniumbromid.

Die Temperatur, bei der die Umsetzung durchgeführt wird, kann in weitem Bereich variiert werden. Die Umsetzung läuft bereits bei Temperaturen von —80°C ab. Die obere Grenze des Temperaturbereiches wird durch den Siedepunkt des verwendeten Lösungsmittels bestimmt. Vorzugsweise arbeitet man bei Temperaturen im Bereich von —20 bis +40°C.

Die folgenden Beispiele erläutern das erfindungsgemäße Verfahren. Die in situ gebildeten Nitriloxide werden hierbei mit gegebenenfalls in 1-Stellung durch Alkyl substituierten Propargylhalogeniden oder -alkoholen zu 1,2-Oxazolen der Formel

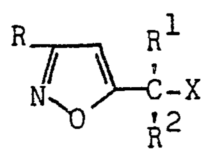

in der $R^1$ und $R^2$ für Wasserstoff oder Alkyl und X für Halogen oder Hydroxyl stehen und R die oben genannten Bedeutungen hat, umgesetzt.

Beispiel 1

Herstellung von 3-Methyl-5-chlormethyl-1,2-oxazol unter Verwendung von Methylnitriloxid

a) durch Umsetzung mit Natriumhypochlorit

37,3 g Propargylchlorid und 26,6 g Acetaldoxim in 200 ml Methylenchlorid werden bei 15—20°C tropfenweise mit 375 g 10 %iger wäßriger Natriumhypochlorit-Lösung versetzt. Danach wird eine Stunde lang bei Raumtemperatur nachgerührt. Die organische Phase wird abgetrennt, die Wasserphase einmal mit 200 ml Methylenchlorid extrahiert. Nach dem Trocknen der vereinigten Methylenchloridphasen über Natriumsulfat wird filtriert, vom Methylenchlorid befreit und destilliert. Man erhält 52,5 g 3-Methyl-5-chlormethyl-1,2-oxazol, entsprechend einer Ausbeute von 89% der Theorie.

Kp. 50—53°C bei 0,4 Torr; $n_D^{25} = 1{,}4810$

b) durch Umsetzung mit Natriumhypobromit

30 g Acetaldoxim werden bei 0°C zu 1 l 3 n Natronlauge gegeben; zu dieser Lösung gibt man bei —5 bis 0°C 45 g Propargylchlorid, gelöst in 200 ml Tetrachlorkohlenstoff, und tropft anschließend 85 g Brom, gelöst in 100 ml Tetrachlorkohlenstoff, zu. Dann wird zwei Stunden lang bei 0°C anschließend über Nacht bei Raumtemperatur nachgerührt. Die dünnschichtchromatographische Analyse ergibt kein Isoxazol. Nach Abtrennen, Trocknen der organischen Phase über Natriumsulfat und Abdestillieren des Lösungsmittels erhält man 1 g eines dunkelbraunen festen Rückstandes.

Beispiel 2

Herstellung von 3-tert.-Butyl-5-chlormethyl-1,2-oxazol unter Verwendung von tert.-Butylnitriloxid

a) durch Umsetzung mit Natriumhypochlorit

Zu 44,7 g Propargylchlorid und 40,5 g Trimethylacetaldoxim in 300 ml Methylenchlorid werden bei 20 bis 25°C 600 g 10%ige Natriumhypochloritlösung, in der 5 Plätzchen Natriumhydroxid gelöst wurden, zugetropft. Das Reaktionsgemisch wird über Nacht bei Raumtemperatur gerührt. Danach wird die organische Phase abgetrennt und die wäßrige Phase mit Methylenchlorid extrahiert; die vereinigten Methylenchloridphasen werden über Natriumsulfat getrocknet. Durch Destillation erhält man 63,1 g 3-tert.-Butyl-5-chlormethyl-1,2-oxazol, entsprechend 91% der Theorie Ausbeute; $n_D^{25} = 1,4747$.

b) durch Umsetzung mit Natriumhypobromit

Zu 28,4 g Trimethylacetaldoxim werden bei Raumtemperatur 570 ml 2 n Natronlauge und nach Abkühlen auf 0°C 22,4 g Propargylchlorid, gelöst in 200 ml Toluol, zugegeben. Dann werden innerhalb von 70 Minuten 45,5 g Brom bei 0°C langsam in das Reaktionsgemisch eingetropft. Danach wird 2 Stunden lang bei 0 bis 10°C, anschließend 1 Stunde lang bei Raumtemperatur nachgerührt. Die organische Phase wird abgetrennt, die wäßrige Phase wird mit Toluol gewaschen, und die vereinigten organischen Phasen werden über Natriumsulfat getrocknet. Nach Abdestillieren des Lösungsmittels erhält man 5 g eines hellen Öls; $n_D^{25} = 1,4845$.

Nach NMR-spektrometrischer Auswertung sind ca. 50—60 Mol.-% an 3-tert.-Butyl-5-chlormethyl-1,2-oxazol vorhanden.

### Beispiel 3

Herstellung von 3-Äthyl-5-chlormethyl-1,2-oxazol unter Verwendung von Äthylnitriloxid

134 g Propargylchlorid und 120 g Propionaldoxim werden bei 15 bis 20°C tropfenweise mit 1200 g 10%iger wäßriger Natriumhypochlorit-Lösung, in der 3 g Natriumhydroxid gelöst wurden, versetzt. Nach einstündigem Nachrühren bei 20°C wird die organische Phase abgetrennt und destilliert. Man erhält 20,5 g 3-Äthyl-5-chlormethyl-1,2-oxazol, entsprechend einer Ausbeute von 93% der Theorie; $K_p = 55$ bis 57°C bei 0,93 mbar; $n_D^{25} = 1,4792$.

### Beispiel 4

Herstellung von 3-Methyl-5-chlormethyl-1,2-oxazol unter Verwendung von Methylnitriloxid

Zu 34 g 70%iger wäßriger Acetaldoximlösung und 37,3 g Propargylchlorid in 150 ml Methylenchlorid werden bei 20°C 49 g tert.-Butylhypochlorit zugetropft. Nach dreistündigem Rühren bei Raumtemperatur werden 150 ml 5%ige Natriumcarbonatlösung zugetropft. Nach weiterem Rühren über eine Stunde bei Raumtemperatur läßt sich 3-Methyl-5-chlormethyl-1,2-oxazol dünnschichtchromatographisch in der organischen Phase nachweisen.

### Beispiel 5

Herstellung von 3-Methyl-5-chlormethyl-1,2-oxazol unter Verwendung von Methylnitriloxid

In eine Lösung von 51 g 70%ige wäßrige Acetaldoxim-Lösung und 74,5 g Propargylchlorid in 100 ml Methylenchlorid werden bei Raumtemperatur 51,5 g Calciumhypochlorit, gelöst mit 3 Plätzchen Natriumhydroxid in 600 ml Wasser, eingetropft. Dann wird über Nacht bei Raumtemperatur gerührt, die organische Phase wird abgetrennt und die wäßrige Phase wird mit Methylenchlorid extrahiert. Aus den vereinigten organischen Phasen erhält man durch Destillation 41,3 g 3-Methyl-5-chlormethyl-1,2-oxazol, entsprechend einer Ausbeute von 52% der Theorie; $n_D^{25} = 1,4800$.

### Beispiel 6

Herstellung von 3-Methyl-5-chlormethyl-1,2-oxazol unter Verwendung von Methylnitriloxid

37,3 g Propargylchlorid, 34 g 70%ige wäßrige Acetaldoximlösung und 1 g Triäthylbenzylammoniumchlorid in 300 ml Toluol werden bei 20 bis 25°C tropfenweise mit 375 g 10%iger Natriumhypochloritlösung, in der 5 Plätzchen Natriumhydroxid gelöst sind, versetzt.

Danach wird über Nacht bei Raumtemperatur nachgerührt. Das Reaktionsgemisch wird mit Natriumchlorid gesättigt, die wäßrige Phase wird abgetrennt und mit Toluol extrahiert. Die vereinigten Toluolphasen werden über Natriumsulfat getrocknet und eingeengt. Durch Destillation gewinnt man 28 g 3-Methyl-5-chlormethyl-1,2-oxazol, entsprechend einer Ausbeute von 55% der Theorie; $n_D^{25} = 1,4818$.

Entsprechend wurden die im folgenden tabellarisch aufgeführten 1,2-Oxazole der Formel

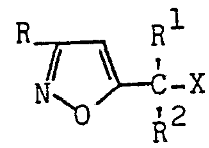

unter Verwendung von Nitriloxiden der Formel R—C≡N→O hergestellt.

| R | $R^1$ | $R^2$ | X | $n_D^{25}$ |
|---|---|---|---|---|
| $CH_3$ | $CH_3$ | H | Cl | 1,4745 |
| $C_2H_5$ | $CH_3$ | H | Cl | 1,4760 |
| $CH_3$ | $CH_3$ | $CH_3$ | Cl | |
| i-$C_3H_7$ | H | H | Cl | 1,4780 |
| $CH_3$ | H | H | I | 1,5505 |
| $C_2H_5$ | H | H | Br | 1,5110 |
| i-$C_3H_7$ | H | $CH_3$ | Cl | 1,4708 |
| $CH_3OCH_2$— | H | $CH_3$ | Cl | 1,4750 |
| $CH_3O$—$CH(CH_3)$— | H | H | Cl | |
| n-$C_4H_9$ | H | H | Cl | 1,4740 |
| n-$C_4H_9$ | $CH_3$ | H | Cl | 1,4701 |
| $CH_3OCH_2$— | H | H | Cl | 1,4800 |
| sec.-$C_4H_9$ | H | $CH_3$ | Cl | 1,4710 |
| n-$C_3H_7$ | H | H | Cl | 1,4768 |
| $ClCH_2$— | H | $CH_3$ | Cl | 1,5002 |
| sec.-$C_4H_9$ | H | H | Cl | 1,4738 |
| n-$C_3H_7$ | H | $CH_3$ | Cl | 1,4712 |
| $ClCH_2$— | $CH_3$ | $CH_3$ | Cl | |
| i-$C_4H_9$ | H | H | Cl | 1,4720 |
| $CH_3$ | H | H | Br | 1,5168 |
| t-$C_4H_9$ | $CH_3$ | H | Cl | 1,4708 |
| i-$C_4H_9$ | H | $CH_3$ | Cl | 1,4684 |
| 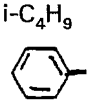 $CH_2$— | $CH_3$ | H | OH | 1,5495 |
| i-$C_4H_9$ | H | $CH_3$ | OH | 1,4558 |
| sec.-$C_4H_9$ | H | $CH_3$ | OH | 1,4700 |
| $CH_3OCH_2$— | H | $CH_3$ | OH | 1,4729 |
| n-$C_3H_7$ | H | $CH_3$ | OH | 1,4693 |

| R | $R^1$ | $R^2$ | X | $n_D^{25}$ |
|---|---|---|---|---|
| $CH_3OCH_2$ | H | H | OH | 1,4778 |
| i-$C_4H_9$ | H | H | OH | 1,4713 |
| i-$C_3H_7$ | H | H | OH | 1,4735 |
| $CH_3$ | H | $CH_3$ | OH | 1,4725 |
| t-$C_4H_9$ | H | H | Cl | 1,4747 |

**Patentanspruch**

Verfahren zur Herstellung von 1,2-Oxazolderivaten durch Umsetzung von Propargylhalogenid oder -alkohol mit in situ hergestellten aliphatischen Nitriloxiden der Formel I

$$R—C{\equiv}N{\rightarrow}O \qquad (I)$$

in der R für einen unverzweigten oder verzweigten, gesättigten aliphatischen Rest steht, der einen oder mehrere Substituenten tragen kann, die nicht mit Hypochlorit reagieren, dadurch gekennzeichnet, daß das verwendete Nitriloxid durch Umsetzung eines Aldoxims der Formel II

$$R—CH{=}NOH \qquad (II),$$

in der R die oben genannte Bedeutung hat, mit Hypochlorit in Gegenwart eines Lösungsmittels bei Temperaturen im Bereich zwischen −80°C und dem Siedepunkt des Lösungsmittels hergestellt wird und das in situ gebildete Nitriloxid der Formel I zu einem 1,2-Oxazolderivat umgesetzt wird.

**Revendication**

Procédé de préparation de dérivés du 1,2-oxazole par réaction d'un halogénure ou alcool propargylique avec des oxydes de nitriles aliphatiques, formés in situ, de la formule I

$$R—C{\equiv}N{\rightarrow}O \qquad (I),$$

dans laquelle R représente un reste aliphatique saturé, ramifié ou non, qui peut porter un ou plusieurs substituants ne réagissant pas avec un hypochlorite, caractérisé en ce que l'oxyde de nitrile employé est préparé par réaction d'un aldoxime de la formule II

$$R—CH{=}NOH \qquad (II),$$

dans laquelle R possède la signification définie, en présence d'un solvant, à des températures comprises entre −80°C et le point d'ébullition du solvant, avec un hypochlorite et l'oxyde de nitrile de la formule I, formé in situ, est ensuite transformé en un dérivé du 1,2-oxazole.

**Claim**

A process for the production of 1,2-oxazole derivatives by reacting propargyl halide or alcohol with aliphatic nitrile oxides, formed in situ, of the formula I

$$R—C{\equiv}N{\rightarrow}O \qquad (I),$$

where R denotes a linear or branched, saturated aliphatic radical which may bear one or more substituents that do not react with hypochlorite, characterized in that the nitrile oxide used is produced by reacting an aldoxime of the formula II

$$R—CH{=}NOH \qquad (II),$$

where R has the above meaning, with hypochlorite in the presence of a solvant at a temperature of from −80°C to the boiling point of the solvent, and the nitrile oxide, formed in situ, of the formula I is reacted to give a 1,2-oxazole derivative.